# EUROPÄISCHE PATENTSCHRIFT

(11) **EP 1 690 268 B1**
(45) Veröffentlichungstag und Bekanntmachung des Hinweises auf die Patenterteilung: **02.06.2010**
(21) Anmeldenummer: 03789095.1
(22) Anmeldetag: 27.11.2003
(51) Int. Cl.: H01F 38/18, A61B 6/00

(54) **COMPUTERTOMOGRAPH MIT BERÜHRUNGSLOSER ENERGIEÜBERTRAGUNG**
COMPUTER TOMOGRAPH WITH CONTACT-FREE ENERGY TRANSFER
TOMODENSITOMETRE A TRANSMISSION SANS CONTACT D'ENERGIE

(43) Veröffentlichungstag der Anmeldung: 16.08.2006
(73) Patentinhaber: Schleifring und Apparatebau GmbH, 82256 Fürstenfeldbruck (DE)
(72) Erfinder: KRUMME, Nils, 82340 Feldafing (DE)
(74) Vertreter: Lohr, Georg
(86) Internationale Anmeldenummer: PCT/EP2003/013354
(87) Internationale Veröffentlichungsnummer: WO 2005/064625

(56) Entgegenhaltungen:
- DE-A- 3 923 525
- DE-A- 19 649 682
- US-A- 5 608 771
- US-A1- 2001 008 552
- PATENT ABSTRACTS OF JAPAN vol. 1997, no. 04, 30. April 1997 (1997-04-30) -& JP 08 336521 A (HITACHI MEDICAL CORP), 24. Dezember 1996 (1996-12-24)

## Beschreibung

### Technisches Gebiet

Die Erfindung betrifft einen Computertomografen mit berührungsloser Energieübertragung. Hierbei erfolgt die Übertragung der von der Röntgenröhre benötigten Energie berührungslos zwischen einer stationären Stromversorgung und der drehend angeordneten Röntgenröhre. Gleichzeitig können weitere Verbraucher wie Detektoren oder Datenerfassungssysteme auf dem drehenden Teil mit versorgt werden.

### Stand der Technik

Bei konventionellen Computertomografen erfolgt die Übertragung elektrischer Energie zwischen der stationär angeordnete Stromversorgung und dem rotierenden Teil mittels mechanischer Schleifringsysteme. Hierbei schleift eine Bürste, vorzugsweise aus Kohlematerial auf einer Schleifbahn, beispielsweise aus Messing. Nachteilig an dieser Anordnung ist die geringe Lebensdauer, die regelmäßigen Wartungsintervalle, in denen die Bürsten ausgetauscht werden müssen und diese durch den Kohleabrieb verursachte Verschmutzung.

Eine Verbesserung ist beispielsweise in der US 4,91 2,735 offenbart. Darin wird das Computertomografen-system wie ein rotierender Übertrager ausgebildet. Auf der stationären Seite ist eine Primärwicklung, die von einer primär angeordneten Wechselstromquelle gespeist wird, angeordnet. Dieser gegenüberliegend ist auf der rotierenden Seite eine Sekundärwicklung angebracht. Zur besseren Verkoppelung zwischen Primärwicklung und Sekundärwicklung sind diese von rotationssymmetrischen Kernen aus weichmagnetischen Materialien umgeben. Diese Vorrichtung ist allerdings nicht zur Übertragung hoher Leistungen im Bereich von 100 Kilowatt, wie sie zur Speisung moderner Röntgenröhre benötigten werden, geeignet. Dies liegt daran, dass der Übertrager aufgrund der unvermeidlichen Luftspalte zwischen der stationären und der rotierenden Seite eine hohe Streuinduktivität aufweist. Diese verhält sich elektrisch wie eine Serieninduktivität und stellt somit eine hohe Serienimpedanz für den zu übertragenen Strom dar, welche die übertragbare Leistung begrenzt.

Eine weitere Verbesserung ist in der US 5,608,771 offenbart. Hier wird die Streuinduktivität des Übertragung mittels einer weiteren Induktivität und einer Kapazität zu einem Resonanzkreis ergänzt. Gleichzeitig wird der Hochspannungsübertrager unmittelbar an die Sekundärwicklung des rotierenden Teiles angeschlossen. Bei dieser Anordnung kann nun eine gewisse Streuinduktivität toleriert werden. Allerdings ist ein sehr hoher Koppelfaktor des Drehübertragers notwendig, da sonst ein zu hoher Stromanteil als Blindstrom durch die Primärwicklung des Übertragers fließen würde. Zudem wäre bei hoher Streuinduktivität kaum eine vernünftige Anpassung an den Hochspannungsübertrager möglich.

Nachteilig an den beiden zitierten Anordnungen ist der hohe Materialeinsatz an teuren, hochpermeablen ferromagnetischen Materialien. So wären bei einer typischen Dimensionierung einige 100 Kilogramm an Eisen bzw. Ferritmaterial notwendig. Diese würden auch das Gesamtgewicht des Computertomografen wesentlich erhöhen. Besonders störend ist die große Masse an dem rotierenden Teil, da hier auch die Lagerung entsprechend tragfähiger ausgestaltet werden muss. Ein weiterer Nachteil ergibt sich in den hohen Anforderungen an die mechanischen Toleranzen in der Drehung zwischen dem rotierenden und dem stationären Teil. So sollte der Luftspalt zwischen der Primärseite im stationären Teil und der Sekundärseite im rotierender Teil idealerweise im Bereich einiger Zehntel Millimeter sein. Die typischen bei Computertomografen realisierbaren Toleranzen liegen jedoch um fast eine Größenordnung höher. Besonders kritisch ist hier der Betrieb bei einem gegenüber der Horizontalachse geneigten rotierenden Teil, da sich hier der rotierenden Teil gegenüber dem stationären Teil aus seiner Normallage verkippt.

In der JP-A-08336521 ist ein induktiver Drehübertrager für Computertomographen offenbart, welcher einen Übertrager aus zwei um den Umfang der Anordnung angeordneten Spulen mit Eisenkernen aufweist.

In der DE 39 23 525 A1 ist ein weiterer induktiver Drehübertrager für Computertomographen mit entlang des Umfangs angeordneten Spulen mit Eisenkernen offenbart.

Die DE 196 49 682 A1 offenbart eine Vorrichtung zur induktiven Signalübertragung, bei der eine reflexionsfrei abgeschlossene Leitung entlang eines Kreisumfangs geführt wird. Zum Signalabgriff ist ein induktiver Koppler vorgesehen.

Die US 2001/0008552 A1 offenbart eine weitere Anordnung zur induktiven Energieübertragung für Computertomographen, wobei auch hier entlang des Umfangs Eisen- bzw. Ferritkerne vorgesehen sind. Allerdings sind hier die Eisen- bzw. Ferritkerne in einzelne Teile unterteilt, welche jeweils wieder eine eigene Teilwicklung aufweisen.

### Darstellung der Erfindung

Der Erfindung liegt die Aufgabe zugrunde, eine Vorrichtung zur kontaktlosen Übertragung elektrischer Energie zwischen dem stationären und dem rotierenden Teil eines Computertomografens gegenüber dem Stand der Technik kostengünstiger zu gestalten und weiterhin derart auszubilden, dass die Masse der gesamten Anordnung reduziert wird und weiterhin größere mechanischen Toleranzen zwischen dem rotierenden Teil und dem stationären Teil zulässig sind.

Eine erfindungsgemäße Lösung dieser Aufgabe ist in den unabhängigen Patentansprüchen angegeben. Weiterbildun-gen der Erfindung sind Gegenstand der abhängigen Ansprüche.

Die erfindungsgemäße Vorrichtung umfasst einen Computertomografen mit einem rotierenden Teil 1 und einem stationären Teil 2. Das stationären Teil umfasst ein Lager 3 zur drehbaren Lagerung des rotierenden Teils 1. Weiterhin ist an dem stationären Teil wenigstens ein Wechselrichter (Inverter) 6 zur Erzeugung eines Wechselstroms vorgesehen. Dieser Wechselstrom weist wenigstens die Grundwelle einer ersten Frequenz auf. Ferner ist eine Leiteranordnung 7 vorhanden, welche von dem Wechselstrom eines oder mehrerer Wechselrichter 6 gespeist wird. Diese Leiteranordnung ist zumindest entlang eines Teils einer Kreisbahn an dem stationären Teil angeordnet. Eine erfindungsgemäße Leiteranordnung besteht zu wesentlichen Teilen aus elektrische Leitern, welche auf einem Träger oder auf Stützen 23 gelagert sind. Eine solche Leiteranordnung ist wesentlich einfacher zu realisieren, als die aus dem Stand der Technik bekannten rotierenden Transformatoren, bei denen an Stelle der einfachen Leiteranordnung primärseitig bereits eine vollständig ausgebildete Primärseite eines Transformators notwendig ist. Diese umfasst neben der Entwicklung und Isolation auch Eisen- bzw. Ferritkerne, welche mit geringen mechanischen Toleranzen zur Ausbildung eines möglichst geringen Luftspalts zwischen Primärseite (stationär) und Sekundärseite (rotierend) gefertigt werden müssen. Zur Auskopplung des elektrischen Stromes aus der Leiteranordnung ist ein induktiver Koppler 8 an dem rotierenden Teil angebracht. Dieser induktiver Koppler 8 weist eine Länge auf, welche kurz gegenüber der Länge der Leiteranordnung 7 ist und wird durch die Bewegung des rotierenden Teils gegenüber dem stationären Teil längs der Leiteranordnung bewegt. Der von dem induktiver Koppler 8 ausgekoppelte Strom kann nun zur Speisung von Verbrauchern wie der Röntgenröhre 4 oder auch eine Detektoranordnung 5 auf dem rotierenden Teil verwendet werden.

In dieser Darstellung wird der Begriff "Strom" im Sinne eines allgemeinen Begriffes für elektrische Energie verwendet. Ebenso könnte stattdessen auch auf die Begriff Spannung bzw. Energie Bezug genommen werden.

Eine weitere Vorrichtung ist ähnlich der oben beschriebenen Vorrichtung ausgebildet. Es sind allerdings Leiteranordnung 7 und Koppler 8 miteinander vertauscht. So ist der Koppler 8 dem stationären Teil 2 zugeordnet und wird von dem Wechselrichter 6 mit Wechselstrom gespeist. Gegenüber dem Koppler 8 beweglich ist die Leiteranordnung 7 auf dem rotierenden Teil 1 angeordnet. Entsprechend kann nun der von der Leiteranordnung 7 ausgekoppelte Strom zur Speisung von Verbrauchern wie der Röntgenröhre 4 oder auch der Detektoranordnung 5 auf dem rotierenden Teil verwendet werden.

Eine besonders vorteilhafte Ausgestaltung der Erfindung sieht eine Leiteranordnung vor, welche 1, 2 oder auch 3 parallel geführte elektrische Leiter (9a, 9b, 9c) umfasst. Weiterhin werden diese Leiter derart von Strömen durchflossen, dass die Summe der Ströme durch alle Leiter an jeder Winkelposition der Leiteranordnung gleich Null ist. Wird die Leiteranordnung beispielsweise durch einen radialen Schnitt an einer beliebigen Stelle durchtrennt und die an dieser Stelle laufenden Ströme gemessen, so ergibt die Summe der Ströme Null. Dies kann beispielsweise dadurch realisiert werden, dass bei einem Zweileitersystem durch eine Leiter der Strom in einer ersten Richtung und durch den zweiten Leiter der Strom mit gleicher Größe in entgegengesetzter Richtung fließt. Bei einem Dreileitersystem könnten die Ströme der drei Leiter bei gleicher Amplitude um jeweils 120 Grad phasenverschoben sein. Durch eine solche Ausgestaltung kann die elektromagnetische Emission der Anordnung wesentlich reduziert werden. Da die Summe der Ströme an jedem Stück der Leiteranordnung gleich Null ist, ist auch das äußere Magnetfeld gleich Null. Um eine gute Symmetrierung zu erreichen kann beispielsweise ein Symmetrierübertrager oder ein Gleichtaktfilter eingesetzt werden. Zur Ansteuerung bei mehreren Leitern eignet sich besonders eine Phase - Shift - Brücke im Wechselrichter.

In einer weiteren vorteilhaften Ausgestaltung der Erfindung weist die Leiteranordnung 7 in Umfangrichtung mehrere Segmente (10a, 10b, 10c) auf. Durch eine solche Segmentierung lassen sich unterschiedliche Arten elektrischer Energie, wie beispielsweise die hohe Leistung zur Speisung der Röntgenröhre und die elektrische Hilfsversorgung durch getrennte Segmente übertragen. Ebenso kann durch Parallelschaltung mehrerer Segmente die gesamte übertragene Leistung erhöht werden. Selbstverständlich können auch mehrere Leiteranordnungen parallel zueinander d. h. beispielsweise in axialer Richtung nebeneinander oder in radialen Richtung ineinander mit entsprechenden Kopplern angeordnet sein.

Eine weitere vorteilhafte Ausgestaltung der Erfindung sieht vor, dass mehrere Koppler 8 vorgesehen sind, wobei zu jedem Zeitpunkt wenigstens ein Koppler im Eingriff mit der Leiteranordnung 7 ist. Der Begriff des Zeitpunkt bezieht sich hier auf eine Drehbewegung des rotierenden Teils 1 gegenüber dem stationären Teil 2. Anders formulierte bedeutet dies, dass an jeder Position des rotierenden Teils 1 wenigstens ein Koppler 8 im Eingriff mit der Leiteranordnung 7 steht. Damit ist zu jedem Zeitpunkt der Bewegung bzw. zu jedem Ort eine Energieübertragung möglich.

In einer weiteren vorteilhaften Ausgestaltung der Erfindung weist wenigstens ein Abgriff 8 weichmagnetisches Material zur Konzentration des magnetischen Flusses auf. So kann der Abgriff beispielsweise mit Eisenmaterial, vorzugsweise in Form von Eisenblechen oder auch Ferritmaterialien versehen sein. Besonders vorteilhaft ist hier der Einsatz von Eisen- bzw. Ferritmaterialien besonders Pulver , welches durch Kunststoff gebunden ist. Wahlweise bzw. zusätzlich kann auch weichmagnetisches Material an der Leiteranordnung 7 vorgesehen sein, um die Verkoppelung zu verbessern.

Eine weitere vorteilhafte Ausgestaltung der Erfindung sieht vor, dass mehrere Wechselrichter 6 vorgesehen sind, wobei jeweils ein Wechselrichter 6 wahlweise einen Leiter 9 und/oder ein Segment 10a, 10b, 10c der Leiteranordnung speist.

In einer weiteren vorteilhaften Ausgestaltung der Erfindung ist wenigstens ein Wechselrichter zur Speisung eines Leiters 9 und/oder eines Segments 10a, 10b, 10c der Leiteranordnung 7 auf oder nahe der jeweiligen Resonanzfrequenz ausgebildet.

In einer anderen vorteilhaften Ausgestaltung der Erfindung werden wahlweise die Leiteranordnung 7 und/oder wenigstens ein Abgriff 8 durch wenigstens eine Kapazität sowie wahlweise eine oder mehrere zusätzliche Induktivitäten zu einem resonanzfähigen Gebilde auf einer vorgegebenen Resonanzfrequenz ergänzt. Besonders günstig ist es, wenn die Anordnung zur Ausbildung einer Serienresonanz durch hinzufügen einer Serienkapazität sowie einer optionalen Serieninduktivität ergänzt wird, falls die Induktivitäten der Leiteranordnung bzw. des Abgriffs nicht hinreichend groß sind. Alternativ hierzu kann die Anordnung auch zur Ausbildung von Parallelresonanzen durch hinzufügen wenigstens einer Parallelkapazität parallel zur Leiteranordnung 7 oder zum Abgriff 8 ausgebildet sein. Der Betrieb des Wechselrichters 6 kann nun an die unterschiedlichen Resonanzbedingungen angepasst werden. Beispielsweise kann eine Regelung der abgegebenen Leistung durch Frequenzvariation des Wechselrichters erfolgen. So wird bei einer Abgabe eines Ausgangstrom des Wechselrichters auf der Resonanzfrequenz sicherlich die maximale Leistung übertragen, während abhängig von der Güte des Resonanzkreises bei Frequenzabweichungen eine geringere Leistung übertragen wird.

Besonders vorteilhaft ist es, bei niedrigen Lastimpedanzen den Wechselrichter 6 auf eine Serienresonanz zu regeln, da diese eine mit sinkender Lastimpedanz steigende Güte aufweist. Bei hohen Lastimpedanzen hingegen ist es vorteilhaft, auf eine Parallelresonanzen regeln, da bei Parallelresonanzen die Güte mit der Lastimpedanz ansteigt. Zweckmäßigerweise ist eine Umschalteinrichtung vorgesehen, welche zunächst die Lastimpedanz, beispielsweise aus dem Verhältnis von Ausgangspannung zu Ausgangstrom des Wechselrichters 6 ermittelt und entsprechend die Frequenzregelung des Wechselrichters auf Parallelresonanz bzw. Serienresonanz konfiguriert.

Grundsätzlich kann ein System aus Wechselrichter 6, Leiteranordnung 7 sowie Abgriff 8 wahlweise zur Leistungsübertragung und gleichzeitig zur Steuerung bzw. Regelung der übertragenen Leistung oder aber ausschließlich zur reinen Leistungsübertragung eingesetzt werden. Wird die übertragene Leistung durch das System gesteuert bzw. geregelt, so ist beispielsweise eine Pulsbreitenmodulation in Pulspaketen des Ausgangssignals oder aber eine Verschiebung der Frequenz abseits der Resonanzfrequenz notwendig. Grundsätzlich verringert sich bei einer Frequenzverschiebung der Wirkungsgrad des Wechselrichters. Ebenso nehmen dabei auch die Emissionen hochfrequenter Signalanteile zu. Alternativ hierzu kann der Wechselrichter immer in einem Betriebspunkt des optimalen Wirkungsgrades bei der Resonanzfrequenz betrieben werden. Um nun die abgegebene Ausgangsgröße zu steuern, ist beispielsweise primärseitig ein weiteres Schaltnetzteil oder eine Leistungsfaktorkorrekturschaltung mit einem in weiten Grenzen einstellbaren Ausgangsbereich notwendig. Wird der Wechselrichter auf der Resonanzfrequenz betrieben, so ist sein Wirkungsgrad sehr hoch, so dass zur Messung des übertragene Stromes nicht der hochfrequente Ausgangstrom gemessen werden muss. Vielmehr kann hierzu die Gleichstromaufnahme des Wechselrichters herangezogen werden.

In einer weiteren vorteilhaften Ausgestaltung der Erfindung ist zumindest ein Wechselrichter 6 zur Erkennung von unterschiedlichen Lastzuständen ausgebildet, so dass dieser erkennen kann falls das diesem zugeordnete Segment der Leiteranordnung 7 nicht im Eingriff mit wenigstens einem Abgriff 8 steht. Entsprechend dieser Erkennung wird nun der Wechselrichter 6 sein Ausgangssignal abschalten bzw. auf eine Leerlauffrequenz steuern.

Weiterhin ist wenigstens ein Wechselrichter 6 zur Abgabe in Signals auf wenigstens einer zweiten Frequenz ausgebildet. Weiterhin ist auf dem rotierenden Teil 1 wenigstens ein frequenzselektives Mittel zur Selektion dieser zweiten Frequenz ausgebildet, so dass dieses nun vorzugsweise die mit der zweiten Frequenz übertragene Energie auskoppelt und hiermit wenigstens einer weiteren Verbraucher, wie beispielsweise einen Steuerrechner oder auch die Detektoranordnung 5 speist.

In einer weiteren vorteilhaften Ausgestaltung der Erfindung ist der Wechselrichter 6 derart ausgebildet, dass das Tastverhältnis seines Ausgangstromes durch eine Steuereinheit gezielt verändert werden kann. Hierbei wird Vorteilhafterweise die Ausgangsfrequenz nicht beeinflusst. Durch die Änderung des Tastverhältnisses ändert sich die spektrale Verteilung des Ausgangstromes. So geht bei einem Tastverhältnis von 50 Prozent, entsprechend einem symmetrischen Ausgangssignal, der Anteil an geradzahligen Vielfachen der Grundfrequenz f0, also 2*f0, 4*f0, 6*f0 etc. im Idealfall gegen Null. Wird das Tastverhältnis zu unsymmetrischen Ausgangsströmen, beispielsweise auch nur geringfügig auf 49 und 48 Prozent oder in größerem Maßstab auf 40 oder 30 Prozent geändert, so steigt die Amplitude der geradzahligen Vielfachen an. Durch selektive Filterung der geradzahligen Vielfachen am rotierenden Teil kann nun selektiv ein bestimmter Anteil der übertragenen Leistung, beispielsweise zur Versorgung kleinerer Verbraucher wie einen Steuerrechner oder der Detektoranordnung ausgekoppelt werden. Vorteilhafterweise ist noch eine Hilfsversorgung vorzusehen, welche für den Fall, dass der zur Abgabe großer Leistung konfigurierte Wechselrichter nicht aktiv ist, weil beispielsweise die Röntgenröhre nicht versorgt werden muss, die Versorgung der kleinerer Verbraucher übernimmt.

Eine weitere vorteilhafte Ausgestaltung der Erfindung sieht wenigstens einen Wechselrichter 6 zur Abgabe eines frequenzmodulierten Ausgangstromes vor. Durch die Modulation der Ausgangsfrequenz verbreitern sich die einzelnen Spektrallinien des Ausgangssignals, wobei sich gleichzeitig ihre Amplitude verringert. Dadurch ergeben sich verbesserte EMV - Eigenschaften des Systems. Die Modulationsfrequenz ist größer oder gleich 100 Hz zu wählen, so dass diese größer als ein Messintervall der gängigen EMV - Normen ist. Weiterhin sollte der Modulationshub, also die Frequenzvariation nur so gering gewählt werden, dass sich keine nennenswerte Schwankungen des Stromes auf der rotierenden Seite ergeben.

### Beschreibung der Zeichnungen

Die Erfindung wird nachstehend ohne Beschränkung des allgemeinen Erfindungsgedankens anhand von Ausführungsbeispielen unter Bezugnahme auf die Zeichnungen exemplarisch beschrieben.
Fig. 1 zeigt beispielhaft eine erfindungsgemäße Vorrichtung in perspektivischer Ansicht.
Fig. 2 zeigt in schematischer Form eine erfindungsgemäße Vorrichtung, wie in Fig. 1 dargestellt, im Schnitt.
Fig. 3 zeigt einen Ausschnitt des oberen Bereichs aus Fig. 2 einer erfindungsgemäßen Vorrichtung.
Fig. 4 zeigt ein elektrisches Blockschaltbild einer beispielhaften erfindungsgemäßen Vorrichtung.
In Fig. 5 ist eine Anordnung mit einem einzelnen Leiter dargestellt.
Fig. 6 zeigt eine Anordnung mit zwei elektrischen Leitern.
Fig. 7 zeigt eine Anordnung mit drei elektrischen Leitern.
Fig. 8 zeigt einen Träger 41 mit darauf angebrachten Platten aus weichmagnetischem Material 44.
Fig. 9 zeigt eine Anordnung mit drei parallel geführten Leitern.
Fig. 10 zeigt eine Anordnung mit segmentierten Leitern.

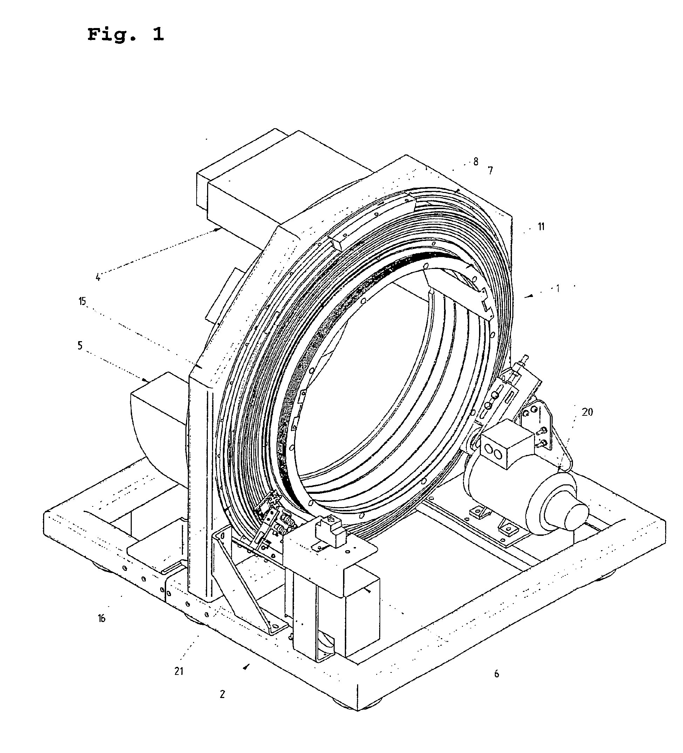

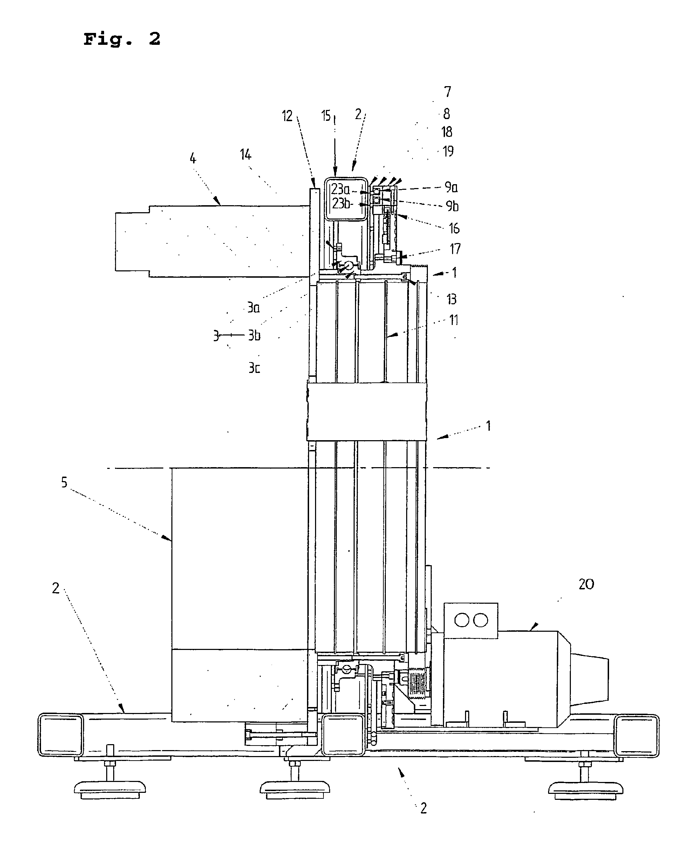

Fig. 1 zeigt beispielhaft eine erfindungsgemäße Vorrichtung in perspektivischer Ansicht. Der Computertomograf (CT-Scanner) besteht aus zwei mechanischen Hauptbestandteilen. Ein stationäres Teil 2 dient als Basis und Träger des ganzen Gerätes, in denen sich das rotierende Teil 1 dreht. Der Patient wird auf einer Liege in der Öffnung des rotierenden Teils positioniert. Zur Lagerung des rotierenden Teils 1 dient das Lager 3 (vgl. Fig.2), welches von dem Hohlprofil 15 des stationären Teils 2 gehalten wird. Dieses Lager ist in der beispielhaften Ausgestaltung als Kugellager ausgeführt. Selbstverständlich sind auch verschiedene andere Lagertypen hierfür einsetzbar. Das Abtastung des Patienten mittels Röntgenstrahlen ist eine Röntgenröhre 4 sowie einen dieser gegenüberliegend angeordneter Detektor 5 vorgesehen. Röntgenröhre 4 und Detektor 5 sind auf dem rotierenden Teil 1 drehbar angeordnet. Zum Antrieb des rotierenden Teiles ist ein Motor 20 vorgesehen. Ein Schleifring 16, welcher an dem rotierenden Teil 1 angebracht ist, dient zusammen mit dem Schleifringabgriff 21, der am stationären Teil 2 befestigt ist, zur Übertragung von Hilfs- und Steuersignalen. So können beispielsweise Sicherheitssignale wie zur Freigabe der Röntgenstrahlung noch über mechanische Schleifkontakte übertragen werden, wie dies derzeit noch von den Sicherheitsstandards gefordert wird. Alternativ hierzu könnte das Aktivierungssignal für die Röntgenröhre auch kontaktlos übertragen werden. Um der Sicherheitsstandards genügen müsste dieses Signal in regelmäßigen Zeitabständen wiederholt werden. Wird das Signal von dem rotierenden Teil nicht in diesen Zeitabständen empfangen, so wird die Röntgenröhre deaktiviert. Die in der mechanischen Konfiguration noch notwendigen zwei Schleifkontakte sind aufgrund der niedrigen Strombelastung nahezu wartungsfrei und verursachen einen wesentlich geringeren Abrieb und somit eine geringere Verschmutzung als die bisher zur Energieübertragung eingesetzten Kontakte. Parallel dazu können mit dieser Anordnung beispielsweise auf kontaktlosem Wege die Bilddaten der Detektoranordnung 5 zum stationären Teil 2 übertragen werden. Zur Energieübertragung, d. h. insbesondere zur Übertragung derjenigen hohen Energie, welche von der Röntgenröhre benötigt wird, ist an dem stationären Teil 2 eine Leiteranordnung 7 vorgesehen, welche von dem Wechselrichter (Inverter) 6 gespeist wird. Der Abgriff der Signale von dieser Leiteranordnung 7 erfolgt mittels eines Kopplers 8 am rotierenden Teil 1. Zur Sicherstellung der Funktion ist wenigstens ein Koppler 8 vorzusehen. Selbstverständlich können auch mehrere Koppler 8 vorgesehen sein. Diese können wahlweise parallel geschaltet sein oder auch zum individuellen Abgriff der Versorgungsenergie für die Röntgenröhre 4, die Detektoranordnung 5 oder andere elektronische Komponenten ausgelegt sein.

Fig. 2 zeigt in schematischer Form eine erfindungsgemäße Vorrichtung, wie in Fig. 1 dargestellt, im Schnitt.

Darin sind die in Fig. 1 dargestellten Teile mit denselben Bezugszeichen gekennzeichnet.

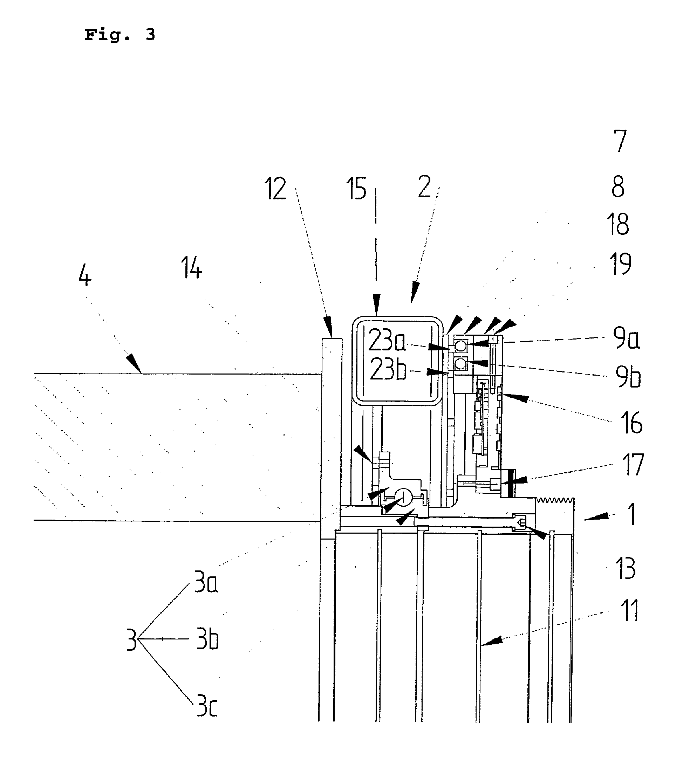

Fig. 3 zeigt einen Ausschnitt des oberen Bereichs aus Fig. 2 einer erfindungsgemäßen Vorrichtung. In dieser Darstellung sind die meisten der zuvor beschriebenen Teile deutlicher zu erkennen. Weiterhin geht der funktionale Zusammenhang der Teile deutlicher hervor. Das stationäre Teil 2 ist in dem dargestellten Bereich zur Erhöhung der Stabilität als Hohlprofil 15 ausgebildet. An diesem ist mittels eines Lagers 3 das rotierende Teil 1 drehbar gelagert. Das Kugellager 3 weist einen äußeren, feststehenden (stationären) Lagerring 3a auf, welcher mittels mehrerer Schrauben 14 an dem stationären Teil 2 befestigt ist. Gegenüber diesem ist mittels der Kugeln 3b der innere Lagerring 3c drehbar gelagert. An diesem ist mittels mehrerer Befestigungsbolzen 13 an einer Seite (im Schnitt rechts dargestellt) ein Zylinder 11 und auf der anderen Seite eine Scheibe 12 befestigt. Die Scheibe 12 trägt die meisten der am rotierenden Teil 1 angebrachten Teile, wie insbesondere die Röntgenröhre 4 sowie in die Detektoranordnung 5. Der Zylinder 11 trägt einen Schleifring 16, welcher mittels der Schrauben 17 am Zylinder 11 befestigt ist. An diesem Schleifring ist hier beispielhaft ein Koppler 8, angebracht, auf einem Trägerelement 18, welches mittels der Schrauben 19 am Schleifring 16 befestigt ist. Dieser Koppler 8 besitzt hier beispielsweise einen U-förmig ausgebildeten Kern aus weichmagnetischem Material, wie beispielsweise Eisen- oder Ferritmaterial. Zur Energieübertragung steht der Koppler 8 im Eingriff mit der Leiteranordnung 7. Die hier dargestellte Leiteranordnung 7 weist beispielhaft zwei parallele Leiter 9a und 9b auf. Diese Leiter sind mittels der Stützen 23a und 23b an dem stationären Teil 2 befestigt. Zur sauberen Positionierung und einfachen Montage der Leiteranordnung 7 ist weiterhin eine Trägerplatte, welche vorteilhafterweise aus weichmagnetischem Material besteht, vorgesehen.

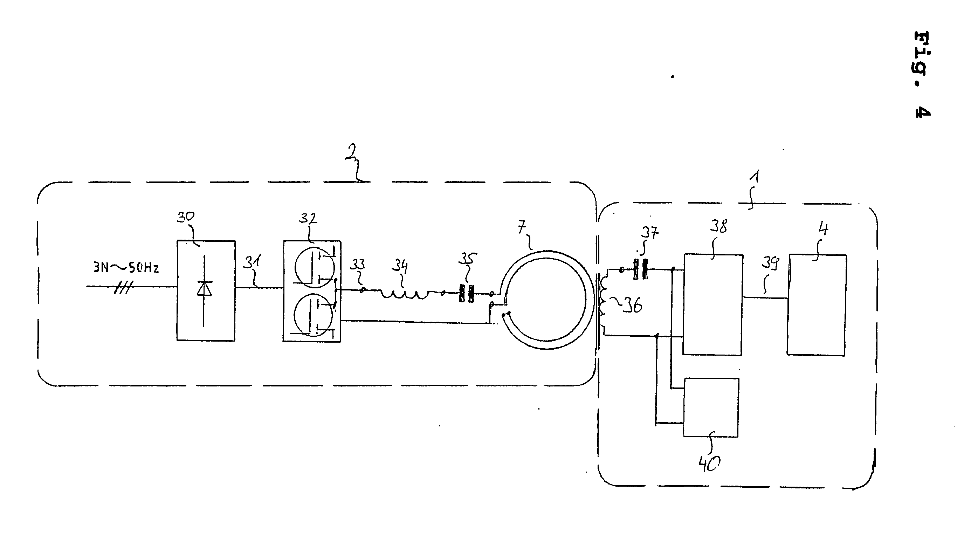

In Fig. 4 ist ein elektrisches Blockschaltbild einer beispielhaften erfindungsgemäßen Vorrichtung dargestellt. Die Versorgung der gesamten Anordnung erfolgt vorteilhafterweise über ein 3-Phasen-Netz mit der gängigen Netzfrequenz, hier beispielhaft 50 Hz. Selbstverständlich ist auch eine zweiphasige oder GleichstromSpeisung möglich. Die Eingangsbeschaltung 30 weist die üblichen Filter sowie eine Gleichrichterschaltung, vorzugsweise mit Leistungsfaktorkorrektur (PFC) auf. Der gleichgerichtete Strom am Ausgang 31 wird mittels eines Wechselrichters (Inverter) 32, welcher typischerweise 2,4 oder mehr Halbleiter-Leistungsschalter aufweist in einen hochfrequente Wechselstrom umgesetzt. Diese Halbleiter-Leistungsschalter können beispielsweise in den bekannten Halbbrücken- oder Vollbrückenschaltungen konfiguriert sein. Als Halbleiterschalter sind bevorzugt IGBTs oder MOSFETs geeignet. Der bevorzugte Frequenzbereich liegt oberhalb der menschlichen Hörschwelle d. h. 20 kHz und reicht bis zu einer oberen Frequenz von ca. einem MHz, bis zu dem moderne Halbleiterschalter für hohe Leistungen wirtschaftlich einsetzbar sind. Der hochfrequente Wechselstrom wird am Ausgang 33 abgegeben und mittels einer Serieninduktivität 34 sowie einer Serienkapazität 35 in die Leiteranordnung 7 eingespeist.

Die Resonanzfrequenz der Anordnung ergibt sich aus der Induktivität 34 sowie der Induktivität der Leiteranordnung 7 zusammen mit der Kapazität 35. Ist die Induktivität der Leiteranordnung 7 hinreichend groß, so kann auch die Induktivität 34 entfallen. Die Induktivität der Leiteranordnung 7 setzt sich zusammen aus einer Induktivität des Leiters selbst sowie der transformierten Induktivität 36 des Kopplers 8 sowie dem Koppelfaktor zwischen der Leiteranordnung 7 und dem Koppler 8. Der von dem Koppler 8 abgegriffene Ausgangstrom kann nun dem Hochspannungsgenerator 38, welcher eine Hochspannung 39 zur Speisung der Röntgenröhre 4 erzeugt zugeführt werden. Parallel zum Hochspannungsgenerator 38 können auch weitere Verbraucher 40 gespeist werden. Der Anschluss an den Koppler 8 kann wahlweise unmittelbar bzw. unter Zwischenschaltung einer Serien-Kapazität 37 erfolgen. Dadurch ergibt sich ein zweiter sekundärseitiger Resonanzkreis. Der Betrieb des Inverters erfolgt zweckmäßigerweise auf bzw. in der Nähe der Resonanzfrequenz des Systems. Eine Steuerung der übertragenen Leistung kann beispielsweise durch Steuerung der Arbeitsfrequenz des Inverters erfolgen, so dass bei einem niedrigeren Leistungsbedarf eine Frequenz abseits der Resonanzfrequenz gewählt wird. Ebenso könnte aber auch eine Steuerung der Leistung durch die Eingangsbeschaltung erfolgen, welche entsprechend dem Leistungsbedarf ihre Gleichspannung 31 einstellt. In diesem Falle kann der nachfolgende Inverter mit optimalem Wirkungsgrad auf der Resonanzfrequenz des Kreises betrieben werden.

Zur Anpassung der Impedanzverhältnisse kann es notwendig sein, an verschiedenen Stellen der Anordnung Übertrager (Transformator) zu schalten. Dies kann insbesondere zwischen dem Wechselrichter 6 und der Leiteranordnung 7, sowie zwischen dem Koppler 8 und der Last notwendig sein. Wird der Wechselrichter zur Speisung des Kopplers 8 eingesetzt, so ein Übertrager zwischen Wechselrichter und Koppler bzw. zwischen Leiteranordnung und Last vorzusehen. Ebenso ist es sinnvoll, insbesondere hinter dem Wechselrichter bzw. an der Leiteranordnung einen Symmetrierübertrager (mit einem weichmagnetischen Kern hoher Güte) bzw. eine Gleichtaktdrossel (Common mode filter) mit einem verlustbehafteten weichmagnetischen Kern einzusetzen.

Zusätzlich kann auf der Lastseite noch eine Regelung der abgegebenen Spannung bzw. des abgegebenen Stromes, wie beispielsweise durch ein Schaltnetzteil vorgesehen sein. So wird sicherlich der Hochspannungsgenerator 38 ebenso wie die Hilfsversorgung 40 eine Regelung der Ausgangspannung aufweisen.

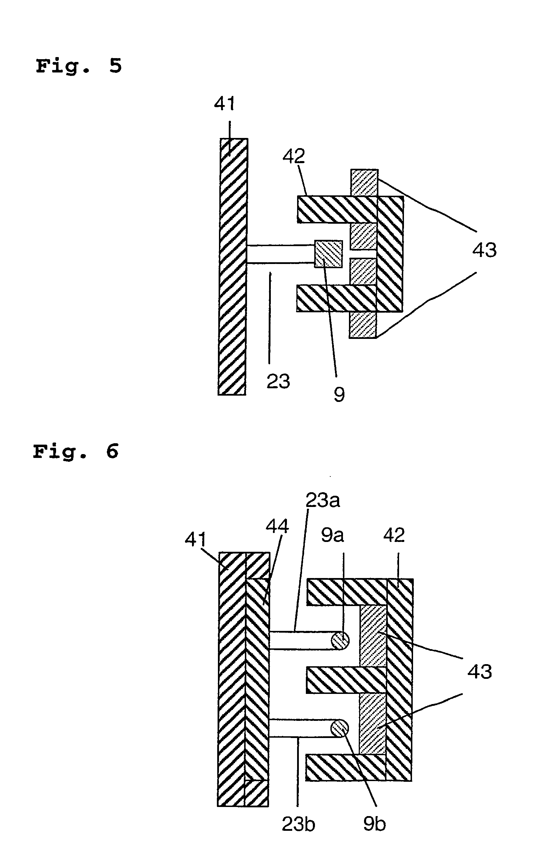

In Fig. 5 ist eine Anordnung mit einem einzelnen Leiter dargestellt. Ein Träger der gesamten Leiteranordnung 41, welcher beispielsweise aus Metall zur Abschirmung oder aber auch aus einem isolierenden Material besteht, trägt einen elektrischen Leiter 9 mittels einer Stütze 23. Zum Abgriff des Stromes läuft entlang des Leiters ein Koppler umfassend einen Kern aus weichmagnetischem Material 42 sowie eine Wicklung 43 zur Auskopplung des elektrischen Stroms.

Fig. 6 zeigt eine Anordnung mit zwei elektrischen Leitern. Die Leiteranordnung umfasst hier einen Träger 41 sowie einer darauf befindliches weichmagnetisches Material 44 zur Führung des Magnetfeldes. Die parallelen elektrischen Leiter 9a und 9b werden mittels der stützen 23a und 23b gelagert. Der Koppler weist hier einen U - förmig ausgestalteten Kern 42 aus weichmagnetischem Material mit wenigstens einer Wicklung 43 auf.

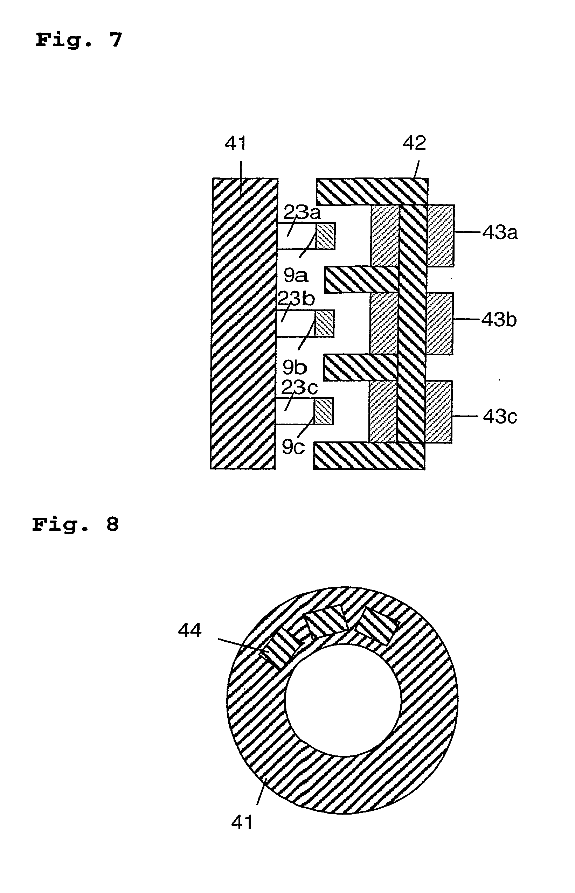

In Fig. 7 ist eine entsprechende Anordnung mit drei Leitern dargestellt. Die Leiter 9a, 9b, 9c sind mittels der Stützen 23a, 23b, 23c auf dem Träger befestigt. Der Koppler weist hier auf dem Kern 42 drei Wicklungen 43a, 43b, 43c auf.

Fig. 8 zeigt einen Träger 41 mit darauf angebrachten Platten aus weichmagnetischem Material 44. Durch eine solche Anordnung kann eine einfache Belegung des Trägers mit vorgefertigten Plattenstücken, welche wahlweise rechteckig oder der Rundung angepasst sind, erfolgen. Zweckmäßigerweise wird nicht nur sowie der Einfachheit halber in der Zeichnung dargestellt ein Teil des Kreisumfangs, sondern der gesamte Kreisumfang mit Plattenstücken belegt.

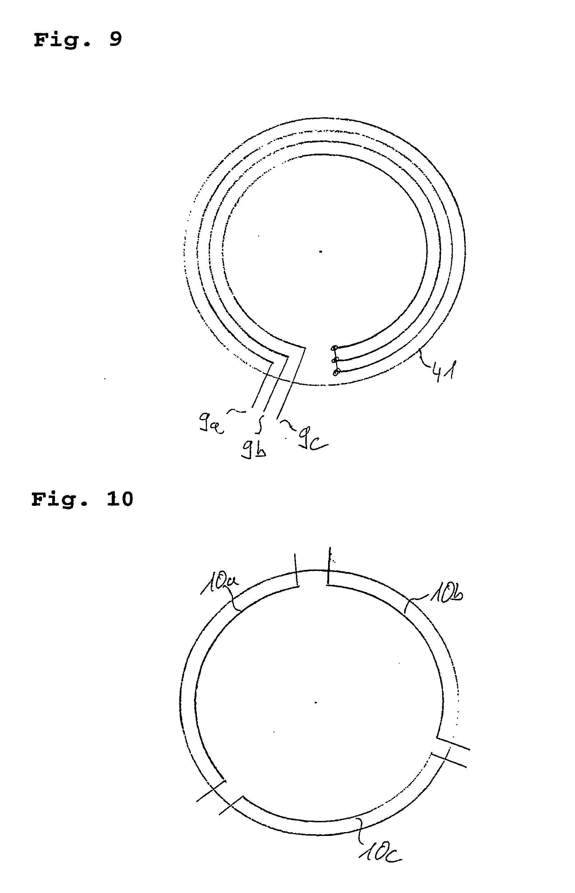

In Fig. 9 ist eine Ausgestaltung der Erfindung mit drei parallel geführten Leitern 9a, 9b, 9c dargestellt. Diese sind auf dem Träger 41 angeordnet. Die offenen Enden werden vom Wechselrichter gespeist. Beispielhaft sind die drei Leiter hier an einem Ende miteinander verbunden. Die Speiseströme vom Wechselrichter sind zweckmäßigerweise jeweils um 120 Grad gegeneinander phasenverschoben. Selbstverständlich ist neben der hier gezeigten Anordnung mit drei Leitern jede andere Anzahl von Leitern einsetzbar.

Fig. 10 zeigt eine Ausgestaltung der Erfindung, bei der die Leiter in Leitersegmente 10a, 10b, 10c unterteilt sind. In Fig.10 sind beispielhaft drei Leitersegmente dargestellt, ebenso können aber auch zwei oder mehr Leitersegmente eingesetzt werden. Die Leitersegmente können jedes für sich aus einem oder auch aus mehreren parallel geführten Leitern bestehen. Die Speisung erfolgt mit einem gemeinsamen Wechselrichter oder individuellen Wechselrichter für einzelne Leitersegmente oder für Gruppen aus Leitersegmenten. Neben einem Frequenzmultiplex ist hier auch ein Ortsmultiplex in der Übertragung möglich. Hierzu werden zweckmäßigerweise mehrere Koppler 8 eingesetzt. Damit können gleichzeitig mehrere Versorgungsströme, beispielsweise für die Röntgenröhre sowie für die Detektoranordnung oder andere Verbraucher getrennt voneinander übertragen werden.

### Bezugszeichenliste

- 1: rotierendes Teil
- 2: stationäres Teil
- 3: Lager
- 4: Röntgenröhre
- 5: Detektoranordnung
- 6: Wechselrichter
- 7: Leiteranordnung
- 8: Koppler
- 9: elektrischer Leiter
- 10: Leitersegmente
- 11: Zylinder
- 12: Scheibe
- 13: Befestigungsbolzen
- 14: Schraube zur Lagerbefestigung
- 15: Hohlprofil
- 16: Schleifring
- 17: Schraube zur Schleifringbefestigung
- 18: Träger des Kopplers
- 19: Schraube zur Trägerbefestigung
- 20: Motor
- 21: Schleifringabgriff
- 23: Stützen
- 30: Eingangsbeschaltung
- 31: Ausgang für Gleichstrom
- 32: Inverter
- 33: Ausgang für HF-Wechselstrom
- 34: Serien-Induktivität, stationäres Teil
- 35: Serien-Kapazität, stationäres Teil
- 36: Induktivität des Kopplers 8
- 37: Serienkapazität, rotierendes Teil
- 38: Hochspannungsgenerator
- 39: Ausgang für Hochspannung
- 40: Hilfsversorgung
- 41: Träger der Leiteranordnung
- 42: Kern aus weichmagnetischem Material
- 43: Wicklung des Kopplers
- 44: weichmagnetisches Material

## Patentansprüche

1. Computertomografen-System umfassend ein rotierendes Teil (1) zur Aufnahme wenigstens einer Röntgenröhre (4) und einer Detektoranordnung (5), sowie ein stationäres Teil (2) umfassend
- ein Lager (3) zur drehbaren Lagerung des rotierenden Teils (1),
- wenigstens einen Wechselrichter (6) zur Erzeugung eines Wechselstromes einer ersten Frequenz,
- eine Leiteranordnung (7), welche von dem Wechselstrom des wenigstens einen Wechselrichters (6) gespeist wird, und
das rotierende Teil (1) wenigstens einen induktiven Koppler (8) zur Auskopplung von Energie aus der Leiteranordnung (7) aufweist, wobei die Energie zur Speisung der Röntgenröhre (4) und/oder der Detektoranordnung (5) dient,
**dadurch gekennzeichnet, dass**
die Leiteranordnung (7) auf einem Träger oder auf Stützen (23) gelagert ist, und der wenigstens eine induktive Koppler (8) eine Länge aufweist, welche kurz gegenüber der Länge der Leiteranordnung (7) ist, und mit einem Teilstück der gesamten Länge der Leiteranordnung (7) in Eingriff steht und aus dieser elektrische Energie auskoppelt.

2. Vorrichtung nach Anspruch 1,
**dadurch gekennzeichnet, dass**
die Leiteranordnung (7) 2 oder 3 parallele Leiter (9a, 9b, 9c) umfasst, welche derart von Strömen durchflossen werden, dass die Summe der Strömen durch alle Leiter an jeder Stelle der Leiteranordnung gleich Null ist.

3. Vorrichtung nach einem der vorhergehenden Ansprüche,
**dadurch gekennzeichnet, dass**
die Leiteranordnung (7) in Umfangrichtung mehrere Segmente (10a, 10b, 10c) aufweist.

4. Vorrichtung nach einem der vorhergehenden Ansprüche,
**dadurch gekennzeichnet, dass**
mehrere Koppler (8) vorgesehen sind, wobei zu jedem Zeitpunkt wenigstens ein Koppler in Eingriff mit der Leiteranordnung (7) ist.

5. Vorrichtung nach einem der vorhergehenden Ansprüche,
**dadurch gekennzeichnet, dass**
wenigstens ein Koppler (8) weichmagnetisches Material zur Konzentration des magnetischen Flusses umfasst.

6. Vorrichtung nach einem der vorhergehenden Ansprüche,
**dadurch gekennzeichnet, dass**
mehrere Wechselrichter (6) zur Speisung je eines Leiters und/oder eines Segmentes der Leiteranordnung (7) vorgesehen sind.

7. Vorrichtung nach einem der vorhergehenden Ansprüche,
**dadurch gekennzeichnet, dass**
zumindest ein Wechselrichter (6) zur Speisung je eines Leiters und/oder eines Segmentes der Leiteranordnung (7) auf oder nahe der jeweiligen Resonanzfrequenz ausgebildet ist.

8. Vorrichtung nach einem der vorhergehenden Ansprüche,
**dadurch gekennzeichnet, dass**
wahlweise wenigstens einige Serienkapazität in Serie zur Leiteranordnung 7 bzw. zum Abgriff 8 und/oder wahlweise wenigstens eine Parallelkapazität parallel zur Leiteranordnung 7 bzw. zum Abgriff 8 geschaltet ist.

9. Vorrichtung nach einem der vorhergehenden Ansprüche,
**dadurch gekennzeichnet, dass**
zumindest ein Wechselrichter (6) zur Erkennung eines Zustandes ausgebildet ist, bei dem die Leiteranordnung (7) bzw. ein Segment der Leiteranordnung (7) nicht in Eingriff mit wenigstens einem Koppler (8) steht und bei fehlenden Eingriff den Wechselrichter abschaltet bzw. auf eine Leerlauffrequenz steuert.

10. Vorrichtung nach einem der vorhergehenden Ansprüche,
**dadurch gekennzeichnet, dass**
zumindest ein Wechselrichter (6) zur Abgabe eines Wechselstroms auf wenigstens einer zweiten Frequenz, zur Speisung von weiteren Verbrauchern ausgebildet ist und wenigstens ein Koppler (8) bzw. die Beschaltung eines Kopplers (8) frequenzselektiv zur Selektion der zweiten Frequenz ausgebildet ist und überwiegend das abgegriffene Signal der zweiten Frequenz wenigstens einem weiteren Verbraucher zuführt.

11. Vorrichtung nach einem der vorhergehenden Ansprüche,
**dadurch gekennzeichnet, dass**
zumindest ein Wechselrichter (6) zur Abgabe eines Wechselstroms mit veränderbarem Tastverhältnis ausgebildet ist und weiterhin auf der rotierenden Seite eine Filtereinheit zur Selektion von Frequenzanteilen mit geradzahligen Vielfachen der ersten Frequenz und zur Speisung wenigstens eines weiteren Verbrauchers mit den selektierten Frequenzanteilen vorgesehen ist.

12. Vorrichtung nach einem der vorhergehenden Ansprüche,
**dadurch gekennzeichnet, dass**
zumindest ein Wechselrichter (6) zur Abgabe eines Wechselstroms mit modulierter Ausgangsfrequenz ausgebildet ist, wobei der Frequenzhub so gering gewählt ist, dass es sich keine nennenswerte Schwankungen in der Amplitude des Ausgangstromes ergibt und gleichzeitig die Modulationsfrequenz größer oder gleich 100 Hz ist.

## Claims

1. Computer tomograph system comprising a rotating part (1) for accommodating at least one X-ray tube (4) and a detector arrangement (5), and also a stationary part (2) comprising:
- a bearing assembly (3) for rotatably supporting the rotating part (1);
- at least one d.c.-a.c. converter (6) for generating an alternating current at a first frequency;
- a conductor arrangement (7) which is supplied with alternating current from the at least one d.c.-a.c. converter (6);
in which the rotating part (1) has at least one inductive coupler (8) for coupling-out energy from the conductor arrangement (7), with the energy serving to feed the X-ray tube (4) and/or the detector arrangement (5);
**characterized in that**
the conductor arrangement (7) is carried on a mount or supports (23), and the at least one inductive coupler (8) is of a length which is short in comparison with the length of the conductor arrangement (7) and is in engagement with a section of the entire length of the conductor arrangement (7) and couples-out electrical energy from the latter.

2. Device according to claim 1,
**characterized in that**
the conductor arrangement (7) comprises 2 or 3 parallel conductors (9a, 9b, 9c) through which currents flow so that the sum of the currents through all conductors is zero at every point of the conductor arrangement.

3. Device according to any one of the preceding claims,
**characterized in that**
the conductor arrangement (7) comprises a plurality of segments (10a, 10b, 10c) along the circumferential direction.

4. Device according to any one of the preceding claims,
**characterized in that**
a plurality of couplers (8) are provided, with at least one coupler being in engagement with the conductor arrangement (7) at any one instant of time.

5. Device according to any one of the preceding claims,
**characterized in that**
at least one coupler (8) comprises magnetically soft material for concentrating the magnetic flux.

6. Device according to any one of the preceding claims,
**characterized in that**
a plurality of d.c.-a.c. converters (6) are provided for feeding each conductor and/or segment of the conductor arrangement (7).

7. Device according to any one of the preceding claims,
**characterized in that**
at least one d.c.-a.c. converter (6) is adapted to feed one conductor and/or one segment of the conductor arrangement (7) at or close to the respective resonance frequency.

8. Device according to any one of the preceding claims,
**characterized in that**
optionally at least one series capacity is connected in series with the conductor arrangement (7) or the tap 8, and/or optionally at least one parallel capacity is connected in parallel with the conductor arrangement 7 or the tap 8.

9. Device according to any one of the preceding claims,
**characterized in that**
at least one d.c.-a.c. converter (6) is adapted to detect a condition in which the conductor arrangement (7) or a segment of the conductor arrangement (7) is not in engagement with at least one coupler (8), and to switch off the d.c.-ac. converter, or to control it to a no-load frequency in case of non-engagement.

10. Device according to any one of the preceding claims,
**characterized in that**
at least one d.c.-a.c. converter (6) is adapted to supply an alternating current of at least one second frequency to feed further consumer loads, and that at least one coupler (8) or the circuitry of a coupler (8) is adapted to be frequency-selective to select the second frequency, and to supply predominantly the tapped-off signal of the second frequency to at least one further consumer load.

11. Device according to any one of the preceding claims,
**characterized in that**
at least one d.c.-a.c. converter (6) is adapted to supply an alternating current at a variable pulse-width repetition rate; and that furthermore a filter unit is provided on the rotating side for selecting frequency components having whole-number multiples of the first frequency, and for feeding at least one further consumer load with the selected frequency components.

12. Device according to any one of the preceding claims,
**characterized in that**
at least one d.c.-a.c. converter (6) is adapted to supply an alternating current at a modulated output frequency, with the frequency sweep being chosen small so that no significant fluctuations of the amplitude of the output current result, and that simultaneously the modulation frequency is higher than or equal to 100 Hz.

## Revendications

1. Système de tomographie assistée par ordinateur comprenant une partie rotative (1) destinée à recevoir au moins un tube à rayons X (4) et une disposition de détecteurs (5), ainsi qu'une partie fixe (2) comprenant
- un palier (3) pour le support rotatif de la partie rotative (1),
- au moins un onduleur (6) pour produire un courant alternatif d'une première fréquence,
- une disposition de conducteurs (7) qui est alimentée par le courant alternatif de l'au moins un onduleur (6), et
la partie rotative (1) présente au moins un coupleur à induction (8) pour découpler de l'énergie à partir de la disposition de conducteurs (7), l'énergie servant à alimenter le tubes à rayons X (4) et/ou la disposition de détecteurs (5),
**caractérisé en ce que** la disposition de conducteurs (7) est supportée sur un support ou sur des consoles (23) et **en ce que** l'au moins un coupleur à induction (8) a une longueur plus courte que la longueur de la disposition de conducteurs (7) et se trouve en prise avec une partie de la longueur totale de la disposition de conducteurs (7) et capte de l'énergie électrique à partir de celle-ci.

2. Dispositif selon la revendication 1, **caractérisé en ce que** la disposition de conducteurs (7) comprend 2 ou 3 conducteurs parallèles (9a, 9b, 9c) qui sont parcourus par des courants de telle façon que la somme des courants parcourant tous les conducteurs soit égale à zéro en tout point de la disposition de conducteurs.

3. Dispositif selon l'une des revendications précédentes, **caractérisé en ce que** la disposition de conducteurs (7) présente plusieurs segments (10a, 10b, 10c) dans le sens de la circonférence.

4. Dispositif selon l'une des revendications précédentes, **caractérisé en ce que** plusieurs coupleurs (8) sont prévus et à tout moment au moins un coupleur étant en prise avec la disposition de conducteurs (7).

5. Dispositif selon l'une des revendications précédentes, **caractérisé en ce qu'**au moins un coupleur (8) comprend un matériau magnétique doux pour la concentration du flux magnétique.

6. Dispositif selon l'une des revendications précédentes, **caractérisé en ce que** plusieurs onduleurs (6) sont prévus pour alimenter chacun un conducteur et/ou un segment de la disposition de conducteurs (7).

7. Dispositif selon l'une des revendications précédentes, **caractérisé en ce qu'**au moins un onduleur (6) est conçu pour alimenter un conducteur et/ou un segment de la disposition de conducteurs (7) à sa fréquence de résonance ou une fréquence proche de celle-ci.

8. Dispositif selon l'une des revendications précédentes, **caractérisé en ce qu'**au moins une capacité série est montée en série avec la disposition de conducteurs (7) ou la prise (8) et/ou au choix, au moins une capacité en parallèle est montée en parallèle avec la disposition de conducteurs (7) ou la prise (8).

9. Dispositif selon l'une des revendications précédentes, **caractérisé en ce qu'**au moins un onduleur (6) est conçu pour reconnaître un état dans lequel la disposition de conducteurs (7) ou un segment de la disposition de conducteurs (7) n'est pas en prise avec au moins un coupleur (8) et arrête l'onduleur ou le fait passer à une fréquence de marche à vide lorsqu'il n'y a pas de prise.

10. Dispositif selon l'une des revendications précédentes, **caractérisé en ce qu'**au moins un onduleur (6) est conçu pour délivrer un courant alternatif à au moins une deuxième fréquence pour alimenter d'autres consommateurs et au moins un coupleur (8) ou le montage d'un coupleur (8) est conçu de façon sélective en fréquence pour la sélection de la deuxième fréquence et le signal de la deuxième fréquence capté est transmis de manière prédominante à au moins un autre consommateur.

11. Dispositif selon l'une des revendications précédentes, **caractérisé en ce qu'**au moins un onduleur (6) est prévu pour délivrer un courant alternatif à taux d'impulsions modifiable et il est prévu en outre du côté rotatif une unité de filtre destinée à sélectionner les composantes de fréquence représentant des multiples pairs de la première fréquence et à alimenter au moins un autre consommateur avec les composantes de fréquence sélectionnées.

12. Dispositif selon l'une des revendications précédentes, **caractérisé en ce qu'**au moins un onduleur (6) est conçu pour délivrer un courant alternatif à fréquence de sortie modulée, le balayage de fréquence étant choisi suffisamment réduit pour qu'il n'y ait pas de fluctuations notables de l'amplitude du courant de sortie et que la fréquence de modulation soit en même temps supérieure ou égale à 100 Hz.
